# EUROPÄISCHE PATENTSCHRIFT

(11) **EP 3 115 073 B1**
(45) Veröffentlichungstag und Bekanntmachung des Hinweises auf die Patenterteilung: **28.03.2018**
(21) Anmeldenummer: 16174597.1
(22) Anmeldetag: 15.06.2016
(51) Int. Cl.: A61M 15/02, A61M 39/10, F16L 27/04, A61M 16/08

(54) **KONNEKTOR FÜR DEN EINSATZ IN EINEM BEATMUNGSSYSTEM**
CONNECTOR FOR USE IN A VENTILATION SYSTEM
CONNECTEUR À ENFICHER DANS UN SYSTÈME DE RESPIRATION

(30) Priorität: 03.07.2015 DE 102015110732
(43) Veröffentlichungstag der Anmeldung: 11.01.2017
(73) Patentinhaber: Röttger-Lanfranchi, Claudia, 91126 Schwabach (DE)
(72) Erfinder: Röttger-Lanfranchi, Claudia, 91126 Schwabach (DE)
(74) Vertreter: Stippl, Hubert

(56) Entgegenhaltungen:
- AU-A1- 2012 258 488
- DE-U1-202013 103 917
- US-A- 5 042 501
- US-A- 5 267 555
- US-A1- 2009 095 288
- US-A1- 2014 373 831

## Beschreibung

### Technologischer Hintergrund

Der Gegenstand der vorliegenden Erfindung betrifft einen Konnektor für den Einsatz in ein Beatmungssystem mit den weiteren Merkmalen des Oberbegriffs des Patentanspruchs 1. Darüber hinaus betrifft die Erfindung ein Beatmungssystem zur Beatmung eines Patienten mit Atemgas mit den weiteren Merkmalen des Oberbegriffs des Patentanspruchs 8. Beatmungsgeräte dienen der Beatmung von Personen mit unzureichender oder ausgesetzter Eigenatmung. Das Atemgas wird meist mit Sauerstoff angereichert, um die Vitalität eines Patienten zu steigern und bei entsprechenden Krankheitsbildern den Heilungsprozess zu fördern.

Die Beatmung erfolgt dabei ausgehend von dem Beatmungsgerät, von dem aus Atemgas durch einen Schlauch zum Patienten weitergeleitet wird. An das Beatmungsgerät kann sich ein Atemgasbefeuchter anschließen, mit dem das Atemgas zum einen auf den erforderlichen Feuchtegrad und zum anderen auf die erforderliche Temperatur erwärmt werden kann. Eine solche Atemgaskonditionierung dient der Vorbereitung des inspirierten Atemgases (Beatmungsgas) für empfindliche Lungen. Auch bei einer Überbrückung der oberen Atemwege durch einen Endotrachealtubus erfolgt eine Atemgaskonditionierung.

Es besteht auch die Möglichkeit, mittels eines Verneblers ein Medikament als Aerosol in feinster Tröpfchenform dem Atemgas und damit den Atemwegen des Patienten zuzuführen. Vernebler werden meist nur zur Verabreichung von Medikamentenaerosolen verwendet.

### Nächstliegender Stand der Technik

Aus der DE 20 2004 004 809 U1 ist ein Atemtherapiegerät mit einem Luftkompressor zur Erzeugung von Luftimpulsen bekannt, das eine Abgabeeinheit zur Zuführung der Luftimpulse an einen Benutzer sowie eine Sauerstoff-Versorgungs-Einrichtung zum konzentrierten Zuführen von Sauerstoff an die Abgabeeinheit aufweist. Zudem ist eine Medikamenten-Zuführung zur Zufuhr von flüssigen Medikamenten an die Abgabeeinheit vorgesehen, welche einen Medikamenten-Vernebler mit einem Medikamenten-Vorratsbehälter und einen mit der Abgabeeinheit sowie dem Medikamenten-Vernebler verbundenen oder verbindbaren Luftstrom-Generator aufweist. Es ist demnach erforderlich, das vernebelte Medikament in den Atemgasstrom einzuführen, sodass es auch in die Atemwege des Patienten gelangt. Insgesamt ist diese Konstruktion relativ aufwendig.

Aus des US 2009/0 095 288 A1 ist eine Beatmungsvorrichtung bekannt, mit welcher ein Anästhetikum in flüssiger Form, von einem Flüssigkeitsreservoir ausgehend, durch eine Leitung in eine Fördereinrichtung weitergeleitet wird und von dort aus durch eine weitere Leitung in eine Kammer eingeleitet wird. Mittels eines Vibrators wird sodann das Fluid durch kleine Löcher in Tröpfchenform in ein Atemgas eingeleitet.

### Aufgabe der vorliegenden Erfindung

Die Aufgabe der vorliegenden Erfindung besteht darin, einen Konnektor für den Einsatz in einem Beatmungssystem zur Verfügung zu stellen, welcher eine Ionisierung des Atemgases ermöglicht und welcher einfach und kostengünstig herzustellen ist.

### Lösung der Aufgabe

Die vorliegende Erfindung wird bei dem gattungsgemäßen Konnektor dadurch gelöst, die Zuführung als ein mit einer chemischen Substanz bestückbarer lonisationsbereich ausgebildet ist, der von dem Atemgas zumindest bereichsweise durchströmbar ist oder der an das zwischen dem ersten und dem zweiten Anschlussstutzen durchgeleitete Atemgas angrenzt, sodass flüchtige Ionen der chemischen Substanz in das Atemgas einleitbar sind. Die Erfindung ermöglicht es auf einfache Weise, dem Atemgas Ionen einer chemischen Substanz, beispielsweise eines Meersalzes oder eines Vitaminpräparates, in geringsten Mengen zuzuführen. Mittels des Konnektors kann dem Atemgas nur eine sehr geringe Konzentration an Ionen der chemischen Substanz zugeführt werden, indem das Atemgas über die chemische Substanz strömt, dort mit den flüchtigen Ionen der Substanz angereichert wird und weiter zum Patienten fließt. Insbesondere die geringe Konzentration an Ionen im Atemgas kann für einen besonders positiven Effekt auf die Beatmung von Vorteil sein. Das ionisierte Atemgas kann aufgrund des osmotischen Effekts besonders gut über die Atmungsorgane aufgenommen werden und die lebenswichtigen Organe mit dem im Blut angereicherten Sauerstoff versorgen. Durch die erhöhte Sauerstoffaufnahme des Blutes können Organe besser funktionieren und der Zellstoffwechsel kann die Konzentration verbessern. Bei der maschinellen Atemtherapie ermöglicht die Erfindung, eine geeignete Substanz über die Atemwege zu verabreichen, die eine Genesung des Patienten beschleunigen soll.

Vorteilhafterweise kann der Ionisationsbereich in die vom ersten zum zweiten Anschlussstutzen oder umgekehrt verlaufende Hauptströmung des Atemgases hineinragen. Auf diese Weise werden die flüchtigen Ionen der chemischen Substanz von der Strömung mitgerissen und weitertransportiert.

Wie oben bereits ausgeführt, kann der Ionisationsbereich auch nur an das zwischen dem ersten und dem zweiten Anschlussstutzen strömende Atemgas angrenzen. Da die Ionen sich ohnehin verflüchtigen, vermischen sie sich auch in diesem Fall mit dem Atemgas. Außerdem dringt das Atemgas auch in den angrenzenden Ionisationsbereich ein und nimmt dort die Ionen auf für den Weitertransport.

Die chemische Substanz kann insbesondere in fester oder fest-flüssiger Form vorliegen. Die Bestückung des Ionisationsbereichs mit der chemischen Substanz ist damit leicht durchführbar. Dabei kann die chemische Substanz auch in unterschiedlicher Form, zum Beispiel als Tabletten, Pulver, Pellets, Granulat, Gel, usw., vorgesehen sein. Als chemische Substanz kann beispielsweise Meersalz oder ein Vitaminpräparat eingesetzt werden. Die zum Beispiel aus dem Meersalz stammenden flüchtigen Ionen können das Atemgas derart ionisieren, dass es einem Meeresklima oder der Luft eines Solestollen gleichkommt. Mittels der Form und Konsistenz sowie der Menge der chemischen Substanz kann die Konzentration der Ionisierung beeinflusst bzw. eingestellt werden. Der Ionisierungsgrad wird darüber hinaus auch von der Atemgasmenge und von ihrer Konditionierung beeinflusst.

In dem Ionisationsbereich ist ein Filter vorgesehen, in oder auf dem die chemische Substanz positionierbar ist. Dieser Filter verhindert ein Herabfallen von etwaig gelösten Teilchen der chemischen Substanz und damit eine Verunreinigung der Atemgasleitung sowie einen Weitertransport derartiger Teilchen mit dem Atemgas. Der Filter kann entweder in seiner Form steif ausgeführt sein, womit er auch leicht handhabbar ist. Der Filter kann auch eine flexible Form aufweisen, indem er zum Beispiel als Beutel ausgebildet ist.

Mit Vorteil kann in dem Ionisationsbereich ein Korbelement vorgesehen sein, in oder auf dem der Filter angeordnet ist. Das Korbelement kann eine zusätzliche Tragfunktion für den Filter bzw. die chemische Substanz übernehmen. Das Korbelement kann zum Beispiel derart ausgestaltet sein, dass der Filter leicht auf das Korbelement einsetzbar ist. Zusätzlich oder alternativ kann das Korbelement auch eine Schutzfunktion übernehmen, indem zum Beispiel Teilchen der chemischen Substanz oder andere Fremdkörper nicht in die Atemgasleitung gelangen können.

Besonders vorteilhaft ist es, wenn es sich bei dem Filter um einen für das Atemgas und die damit transportierten Ionen durchlässigen Mikrofilter handelt. Insbesondere kann es sich um einen Mikrofilter handeln, wie er auch in der Blutwäsche eingesetzt wird. Der Durchfluss des Atemgases soll dadurch kaum beeinträchtigt werden.

Der Ionisationsbereich kann in einem nach außen ragenden Aufsatz, der sich an dem Konnektor zwischen erstem und zweitem Atemgasstutzen befindet, angeordnet sein, wobei der Aufsatz zum Innenraum des Konnektors offen ist. In diesen Aufsatz kann sodann der Filter mit der chemischen Substanz eingesetzt werden. Der Innenbereich des Aufsatzes kann zum Beispiel durch eine vorspringende Stufe derart geformt sein, dass der eingesetzte Filter sogleich in der korrekten Höhe fixiert ist. Der Konnektor kann zusammen mit dem angeformten Aufsatz als Kunststoffteil im Spritzgussverfahren hergestellt werden.

Der Filter oder das Korbelement kann auch an einem nach außen ragenden Vorsprung oder T-Stück zwischen erstem und zweitem Anschlussstutzen angeordnet sein. Der Konnektor kann auf diese Weise relativ einfach gestaltet werden und ist als Kunststoffteil im Spritzgussverfahren (mit Ausnahme des Filters) herstellbar. Der nach außen ragende Vorsprung bzw. das obere stirnseitige Ende des T-Stücks können dabei als Anlage für den einzusetzenden Filter bzw. Mikrofilter dienen.

Zweckmäßigerweise kann der Aufsatz oder der Filter oder das Korbelement einen Deckel zum Öffnen und Verschließen des Ionisationsbereichs aufweisen. Dadurch ist auch eine Kontrolle der chemischen Substanz möglich, ohne dass der komplette Filter herausgenommen werden muss.

Zusätzlich kann eine Pumpe vorgesehen sein, die die Einleitung der Ionen von der chemischen Substanz in das Atemgas unterstützt und/oder steuert. Der Anschluss zur Pumpe kann im Bereich des Deckels vorgesehen sein. Dabei kann die Pumpe in bestimmten Zeitabschnitten (zum Beispiel jede Stunde oder Minute) einen Druckaufbau im Ionisationsbereich bzw. auf die chemische Substanz ausüben, sodass die Ionisation des Atemgases in den entsprechenden Zeitabständen verstärkt erfolgt. Die Pumpe kann aber auch kontinuierlich arbeiten, sodass laufend eine erhöhte Ionisation erfolgt bzw. mehr flüchtige Ionen in das Atemgas eingeleitet werden. Bei der Pumpe kann es sich zum Beispiel um eine Membranpumpe handeln.

Statt der Pumpe kann auch eine andere Aktivierung der Ionisation der chemischen Substanz, zum Beispiel auf elektronischem Wege, erfolgen.

Der Konnektor kann als Einweg- oder Mehrwegprodukt ausgebildet sein. Als Einwegprodukt kann er zum Beispiel aus Polypropylen bestehen, als Mehrwegprodukt zum Beispiel aus Polycarbonat, sodass er autoklavierbar bzw. sterilisierbar ist.

Das erfindungsgemäße Beatmungssystem zur Beatmung eines Patienten mit Atemgas umfasst ein Beatmungsgerät, einen Schlauch, welcher Atemgas vom Beatmungsgerät zum Patienten leitet, wobei in dem Schlauch ein Konnektor mit mindestens einem der Merkmale, wie sie oben beschrieben sind, eingesetzt ist. Zusätzlich können weitere Bestandteile, wie zum Beispiel ein Befeuchter, vorgesehen sein, um das Atemgas zu erwärmen und mit Feuchtigkeit zu versorgen.

### Beschreibung der Erfindung anhand von Ausführungsbeispielen

Eine zweckmäßige Ausgestaltung des erfindungsgemäßen Konnektors sowie des Beatmungssystems ist anhand von Zeichnungen näher erläutert. Es zeigen:
- Fig. 1: ein stark vereinfachtes Blockschaltbild einer beispielhaften Ausgestaltung des erfindungsgemäßen Beatmungssystems;
- Fig. 2: eine perspektivische Darstellung des Konnektors ohne eingesetzten Filter;
- Fig. 3: eine perspektivische Darstellung des Konnektors mit eingesetztem Filter;
- Fig. 4: eine perspektivische Darstellung des Konnektors mit eingesetztem Filter und Deckel;
- Fig. 5: eine Schnittdarstellung des Konnektors entlang der Linie B-B gemäß Fig. 6;
- Fig. 6: eine Seitenansicht des Konnektors;
- Fig. 7: eine Schnittdarstellung des Konnektors entlang der Linie A-A gemäß Fig. 8 sowie
- Fig. 8: eine Draufsicht auf den Konnektor.

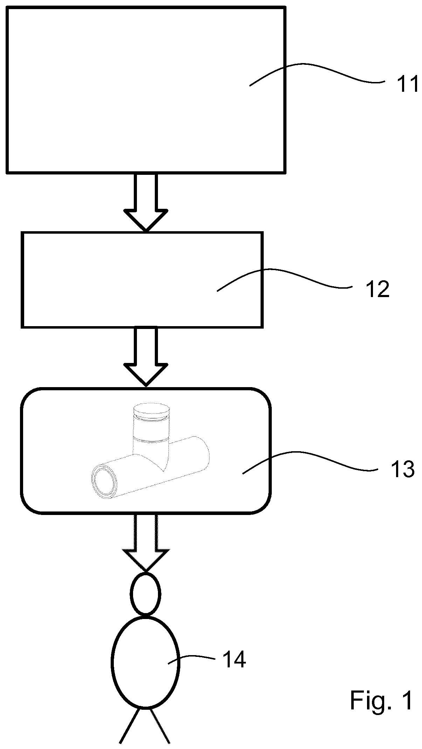

Das Blockschaltbild gemäß Fig. 1 zeigt die wesentlichen möglichen Bestandteile eines Beatmungssystems für einen Patienten, der mit Bezugsziffer 14 versehen ist. Es umfasst ein Beatmungsgerät 11, von welchem das Atemgas in das Schlauchsystem eingeleitet wird. Es folgt ein Befeuchter 12 oder auch Atemgaskonditioniereinrichtung genannt, die oder der dazu dient, dass das von dem Beatmungsgerät 11 ankommende Beatmungsgas auf die gewünschte Temperatur sowie relative Feuchtigkeit bringt. Üblicherweise wird das Beatmungsgas derart konditioniert, dass es eine Temperatur von 37°C und eine relative Feuchtigkeit von 100% aufweist.

Zur Verbindung des Befeuchters 12 mit dem Patienten 14 ist ein Schlauchsystem 13 vorgesehen, welches nicht im Einzelnen beschrieben wird. Das Schlauchsystem 13 umfasst einen Konnektor 1, der symbolisch dargestellt ist. Mittels des Konnektors 1, der als Zwischenstück bei beheizten oder nicht beheizten Patientenschlauchsystemen eingesetzt wird, kann das Atemgas ionisiert werden, was im Folgenden näher erläutert wird.

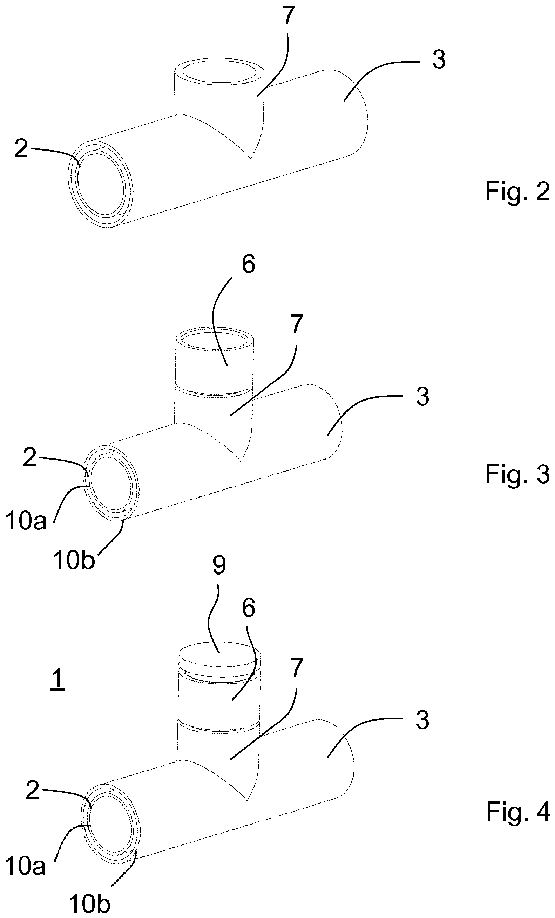

Der in den Fig. 4 bis 8 in dieser Ausführungsvariante vollständig dargestellte Konnektor 1 weist einen ersten Anschlussstutzen 2 sowie einen zweiten Anschlussstutzen 3 zum Einsatz in eine Atemgasleitung und zur Durchleitung des Atemgases auf. Die Atemgasleitung bzw. der Atemgasschlauch selbst ist in den Figuren nicht dargestellt. Der Konnektor 1 weist einen mit einer chemischen Substanz 5 bestückbaren Ionisationsbereich 4 auf, der zwischen erstem 2 und zweitem Anschlussstutzen 3 angeordnet ist. Der Ionisationsbereich 4 kann von dem Atemgas zumindest bereichsweise durchströmbar sein und/oder an die zwischen dem ersten 2 und dem zweiten Anschlussstutzen 3 laufende Hauptströmung des Atemgases angrenzen, sodass flüchtige Ionen der chemischen Substanz 5 in das Atemgas eingeleitet werden. Als chemische Substanz kann zum Beispiel Meeressalz oder ein Vitaminpräparat eingesetzt werden. Das mit den flüchtigen Ionen der chemischen Substanz 5 vermischte Atemgas kann von dem Patienten 14 durch den osmotischen Effekt besonders gut über die Atmungsorgane aufgenommen werden. Die Genesung des Patienten 14 kann damit beschleunigt werden. Vorteilhafterweise können durch diese Anordnung nur die ohnehin flüchtigen Ionen in das Atemgas eingeleitet werden, sodass die Konzentration der Ionisation relativ gering ist.

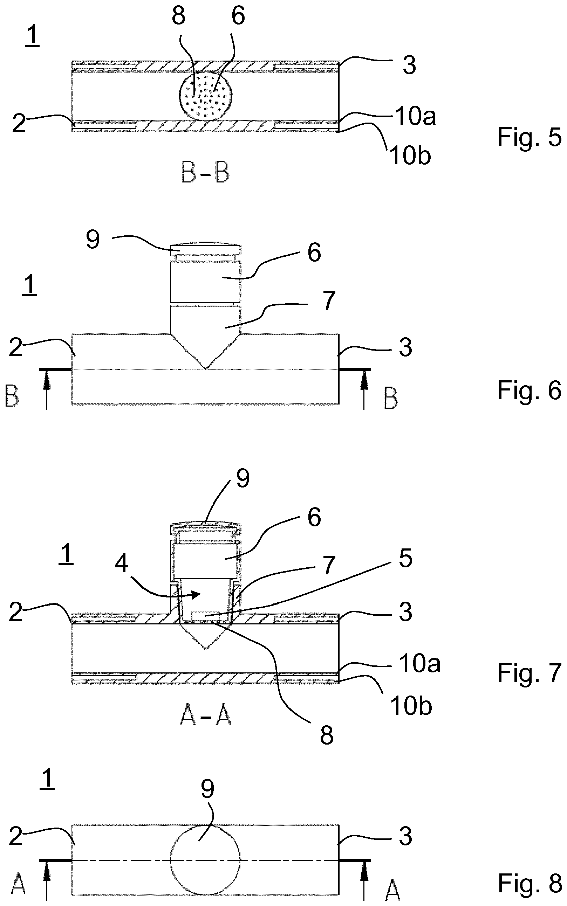

Wie aus der Schnittdarstellung gemäß Fig. 7 deutlich hervorgeht, grenzt der lonisationsbereich 4 bei dieser Ausführungsvariante an das zwischen dem erstem 2 und zweitem Anschlussstutzen 3 strömende Atemgas an. Durch den Filter 6 gelangen die Ionen in die Atemgasleitung zwischen erstem und zweitem Anschlussstutzen 2, 3. Das Atemgas kann auch in den Ionisationsbereich 4 eindringen und sich dort mit den Ionen vermischen, wobei das ionisierte Gas sodann wieder in den Hauptstrom des Atemgases zurückdiffundiert und von dort aus zum Patienten 14 transportiert wird.

Alternativ zu der in Fig. 7 dargestellten Ausführungsvariante kann der Ionisationsbereich 4 und/oder die chemische Substanz 5 direkt in den Hauptstrom des Atemgases hineinragen.

Die chemische Substanz kann vorteilhafterweise in fester, flüssiger oder fest-flüssiger Form vorliegen und in den Konnektor eingesetzt werden. Dabei kann die chemische Substanz zum Beispiel in der Form von Tabletten, Pulver, Pellets, Granulat, Gel und dergleichen vorgesehen sein. In diesen Formen und Konsistenzen ist die chemische Substanz auch gut handhabbar.

In dem Ionisationsbereich 4 ist ein Filter 6 vorgesehen, auf dem die chemische Substanz 5 in Form eines Pellets positioniert ist (siehe Fig. 7). Der Filter 6 verhindert, dass Teilchen der chemischen Substanz 5 in die Atemgasleitung des Konnektors 1 fallen können und mit dem Atemgas weitertransportiert werden.

Der Filter 6 weist nur in seinem Boden 8 durchlässige Filterporen auf (siehe Fig. 5 und 7). Es wird jedoch der gesamte Filtereinsatz (siehe Fig. 3, 6 und 7) als Filter 6 bezeichnet.

Zusätzlich kann in dem Ionisationsbereich 4 ein (in den Zeichnungsfiguren nicht dargestelltes) Korbelement vorgesehen sein, in oder auf dem der Filter angeordnet ist. Das Korbelement kann der Positionierung des Filters dienen und gleichzeitig einen zusätzlichen Schutz darstellen, dass keine Fremdkörper in die Atemgasleitung gelangen können.

Bei dem Filter 6 kann es sich um einen für das Atemgas und die damit transportierten Ionen durchlässigen Mikrofilter handeln. Der Mikrofilter erlaubt damit eine Vermischung der flüchtigen Ionen mit dem Atemgas, verhindert aber ein Eintreten von Partikeln der chemischen Substanz, die größer als die betreffenden Ionen sind.

Der Filter 6 kann an einem nach außen ragenden T-Stück 7, welches sich zwischen dem ersten 2 und zweiten Anschlussstutzen 3 befindet, angeordnet sein. Wie aus Fig. 7 hervorgeht, ist der untere Abschnitt des eingesetzten Filters 6 konisch ausgebildet, sodass er in dem ebenfalls konisch ausgebildeten T-Stück 7 bis zu einem bestimmten Bereich einsetzbar ist und sodann in dieser Position fixiert ist.

An seiner Oberseite kann der Filter 6 einen Deckel 9 zum Öffnen und Verschließen des Ionisationsbereichs bzw. des Filters 6 aufweisen. Die gesamte Beatmungsanordnung ist damit nach außen abschließbar.

Der jeweilige Anschlussstutzen 2, 3 weist zwei konzentrische Anschlussringe, nämlich einen inneren Anschlussring 10a sowie einen äußeren Anschlussring 10b auf. Diese Anschlussringe 10a, 10b dienen zum Anschluss an unterschiedliche Schlauchgrößen mit unterschiedlichem Schlauchdurchmesser. So kann ein Schlauch mit einem geringen Schlauchdurchmesser (zum Beispiel 10 mm) innerhalb des inneren Anschlussrings 10a eingeschoben werden, ein Schlauch mit einem mittleren Durchmesser (zum Beispiel 15 mm) kann zwischen dem inneren Anschlussring 10a und dem äußeren Anschlussring 10b eingeschoben werden und ein Schlauch mit einem größeren Durchmesser (zum Beispiel 22 mm) kann über den äußeren Anschlussring 10b übergeschoben werden. Die jeweiligen Schlauchgrößen sind für unterschiedliche Patientengruppen, insbesondere Säuglinge, Kinder und Erwachsene, bestimmt.

Im Bereich des Deckels kann auch der Anschluss einer (nicht dargestellten) Pumpe vorgesehen sein, die die Einleitung der Ionen in das Atemgas unterstützt und/oder steuert. Die Konzentration des Ionen im Atemgas kann dadurch verändert werden und an die Bedürfnisse des Patienten 14 angepasst werden.

### BEZUGSZEICHENLISTE

- 1: Konnektor
- 2: erster Anschlussstutzen
- 3: zweiter Anschlussstutzen
- 4: Ionisationsbereich
- 5: chemische Substanz
- 6: Filter
- 7: T-Stück
- 8: Boden
- 9: Deckel
- 10a: innerer Anschlussring
- 10b: äußerer Anschlussring
- 11: Beatmungsgerät
- 12: Befeuchter
- 13: Schlauchsystem mit Konnektor
- 14: Patient

## Patentansprüche

1. Konnektor für den Einsatz in ein Beatmungssystem mit
einem ersten (2) und einem zweiten Anschlussstutzen (3) zum Einsatz in eine Atemgasleitung und zur Durchleitung des Atemgases und
einer im Bereich zwischen erstem (2) und zweitem Anschlussstutzen (3) angeordneten Zuführung zur Einleitung eines chemischen Stoffs in das Atemgas,
**dadurch gekennzeichnet, dass**
die Zuführung als ein mit einer chemischen Substanz (5) in fester oder fest-flüssiger Form bestückbarer Ionisationsbereich (4) ausgebildet ist, der von dem Atemgas zumindest bereichsweise durchströmbar ist oder der an das zwischen dem ersten (2) und dem zweiten Anschlussstutzen (3) durchgeleitete Atemgas angrenzt, sodass flüchtige Ionen der chemischen Substanz (5) in das Atemgas einleitbar sind,
in dem Ionisationsbereich (4) ein Filter (6) vorgesehen ist, in oder auf dem die chemische Substanz (5) positionierbar ist.

2. Konnektor nach Anspruch 1, **dadurch gekennzeichnet, dass**
der Ionisationsbereich (4) in die vom ersten (2) zum zweiten Anschlussstutzen (3) oder umgekehrt laufende Strömung des Atemgases hineinragt.

3. Konnektor nach mindestens einem der vorhergehenden Ansprüche,
**dadurch gekennzeichnet, dass**
in dem Ionisationsbereich (4) ein Korbelement vorgesehen ist, in oder auf dem der Filter (6) angeordnet ist.

4. Konnektor nach mindestens einem der vorhergehenden Ansprüche,
**dadurch gekennzeichnet, dass**
es sich bei dem Filter (6) um einen für das Atemgas und die damit transportierten Ionen durchlässigen Mikrofilter handelt.

5. Konnektor nach mindestens einem der vorhergehenden Ansprüche,
**dadurch gekennzeichnet, dass**
der Ionisationsbereich (4) in einem nach außen ragenden Aufsatz angeordnet ist, der zum Innenraum des Konnektors (1) offen ist.

6. Konnektor nach mindestens einem der vorhergehenden Ansprüche,
**dadurch gekennzeichnet, dass**
der Filter (6) oder das Korbelement an einem nach außen ragenden Vorsprung oder T-Stück (7) zwischen erstem (2) und zweitem Anschlussstutzen (3) angeordnet ist.

7. Konnektor nach mindestens einem der vorhergehenden Ansprüche,
**dadurch gekennzeichnet, dass**
der Aufsatz oder der Filter (6) oder das Korbelement einen Deckel (9) zum Öffnen und Verschließen des Ionisationsbereichs (4) aufweist.

8. Beatmungssystem zur Beatmung eines Patienten mit Atemgas mit einem Beatmungsgerät (11),
einem Schlauch, welcher Atemgas vom Beatmungsgerät (11) zum Patienten leitet,
**dadurch gekennzeichnet, dass**
in dem Schlauch ein Konnektor (1) gemäß mindestens einem der Ansprüche 1 bis 7 eingesetzt ist.

9. Beatmungssystem nach Anspruch 8, **dadurch gekennzeichnet, dass**
eine Pumpe vorgesehen ist, die die Einleitung der Ionen in das Atemgas unterstützt und/oder steuert.

## Claims

1. Connector for insertion into a ventilation system,
with
a first nozzle (2) and second nozzle (3) for insertion into a respiratory gas line and for passage of the respiratory gas, and
a feed arranged in the region between first nozzle (2) and second nozzle (3) and serving to introduce a chemical substance into the respiratory gas,
**characterized in that** the feed is designed as an ionization region (4) which can be equipped with a chemical substance (5) in solid or liquid form and through at least part of which the respiratory gas flows, or which borders the respiratory gas conveyed between the first nozzle (2) and the second nozzle (3), such that volatile ions of the chemical substance (5) can be introduced into the respiratory gas, a filter (6) being provided in the ionization region (4), in or on which filter (6) the chemical substance (5) can be positioned.

2. Connector according to Claim 1, **characterized in that** the ionization region (4) projects into the flow of the respiratory gas running from the first nozzle (2) to the second nozzle (3) or vice versa.

3. Connector according to at least one of the preceding claims, **characterized in that** a basket element, in or on which the filter (6) is arranged, is provided in the ionization region (4).

4. Connector according to at least one of the preceding claims, **characterized in that** the filter (6) is a microfilter that is permeable to the respiratory gas and to the ions transported with the latter.

5. Connector according to at least one of the preceding claims, **characterized in that** the ionization region (4) is arranged in an outwardly protruding attachment, which is open towards the interior of the connector (1).

6. Connector according to at least one of the preceding claims, **characterized in that** the filter (6) or the basket element is arranged on an outwardly protruding projection or T-piece (7) between first nozzle (2) and second nozzle (3).

7. Connector according to at least one of the preceding claims, **characterized in that** the attachment or the filter (6) or the basket element has a cover (9) for opening and closing the ionization region (4).

8. Ventilation system for ventilating a patient with respiratory gas, said system having
a ventilator (11),
a tube which conveys respiratory gas from the ventilator (11) to the patient,
**characterized in that** a connector (1) according to at least one of Claims 1 to 7 is inserted in the tube.

9. Ventilation system according to Claim 8,
**characterized in that** a pump is provided which assists and/or controls the introduction of the ions into the respiratory gas.

## Revendications

1. Connecteur pour l'utilisation dans un système respiratoire, comprenant
un premier (2) et un deuxième (3) raccord pour l'insertion dans une conduite de gaz respiratoire et pour guider le gaz respiratoire et
une conduite d'alimentation disposée dans la région entre le premier (2) et le deuxième (3) raccord, pour introduire une substance chimique dans le gaz respiratoire,
**caractérisé en ce que**
la conduite d'alimentation est réalisée sous forme de région d'ionisation (4) pouvant être chargée avec une substance chimique (5) sous forme solide ou solide-liquide, qui peut être parcourue au moins en partie par le gaz respiratoire, ou qui est adjacente au gaz respiratoire circulant entre le premier (2) et le deuxième raccord (3), de telle sorte que des ions volatils de la substance chimique (5) puissent être introduits dans le gaz respiratoire, un filtre (6) étant prévu dans la région d'ionisation (4), dans ou sur lequel peut être positionnée la substance chimique (5).

2. Connecteur selon la revendication 1, **caractérisé en ce que** la région d'ionisation (4) pénètre dans l'écoulement du gaz respiratoire s'écoulant depuis le premier raccord (2) jusqu'au deuxième raccord (3) ou inversement.

3. Connecteur selon au moins l'une quelconque des revendications précédentes, **caractérisé en ce que** dans la région d'ionisation (4) est prévu un élément de panier dans ou sur lequel est disposé le filtre (6).

4. Connecteur selon au moins l'une quelconque des revendications précédentes, **caractérisé en ce que** le filtre (6) est un microfiltre perméable pour le gaz respiratoire et les ions transportés par celui-ci.

5. Connecteur selon au moins l'une quelconque des revendications précédentes, **caractérisé en ce que** la région d'ionisation (4) est disposée dans une garniture faisant saillie vers l'extérieur, qui est ouverte vers l'espace intérieur du connecteur (1).

6. Connecteur selon au moins l'une quelconque des revendications précédentes, **caractérisé en ce que** le filtre (6) ou l'élément de panier est disposé au niveau d'une saillie ou d'une pièce en T (7) faisant saillie vers l'extérieur, entre le premier (2) et le deuxième (3) raccord.

7. Connecteur selon au moins l'une quelconque des revendications précédentes, **caractérisé en ce que** la garniture ou le filtre (6) ou l'élément de panier présente un couvercle (9) pour ouvrir et fermer la région d'ionisation (4).

8. Système respiratoire pour la respiration d'un patient avec un gaz respiratoire, comprenant un appareil respiratoire (11),
un tuyau, qui conduit le gaz respiratoire depuis l'appareil respiratoire (11) jusqu'au patient,
**caractérisé en ce**
**qu'**un connecteur (1) selon au moins l'une quelconque des revendications 1 à 7 est inséré dans le tuyau.

9. Système respiratoire selon la revendication 8,
**caractérisé en ce**
**qu'**il est prévu une pompe qui supporte et/ou commande l'introduction des ions dans le gaz respiratoire.
